(19) **Europäisches Patentamt**

**European Patent Office**

**Office européen des brevets**

(11) **EP 1 693 084 A1**

(12) **EUROPEAN PATENT APPLICATION**
published in accordance with Art. 158(3) EPC

(43) Date of publication:
**23.08.2006 Bulletin 2006/34**

(21) Application number: **04820259.2**

(22) Date of filing: **09.12.2004**

(51) Int Cl.:
*A61P 3/04* (2006.01)      *A61P 3/06* (2006.01)
*A61P 43/00* (2006.01)      *A23L 1/30* (2006.01)
*A23L 2/00* (2006.01)      *C12N 9/99* (2006.01)

(86) International application number:
**PCT/JP2004/018369**

(87) International publication number:
**WO 2005/056034 (23.06.2005 Gazette 2005/25)**

(84) Designated Contracting States:
**AT BE BG CH CY CZ DE DK EE ES FI FR GB GR HU IE IS IT LI LT LU MC NL PL PT RO SE SI SK TR**

(30) Priority: **10.12.2003 JP 2003412188**

(71) Applicant: **RIKEN VITAMIN CO., LTD.**
**Chiyoda-ku,**
**Tokyo 101-8370 (JP)**

(72) Inventors:
• **FUNAYAMA, Katsura**
  **Asaka-shi, Saitama 3510011 (JP)**
• **KAHARA, Takashi**
  **Tokyo 165-0025 (JP)**

• **TANAKA, Minoru**
  **Itabashi-ku, Tokyo1740072 (JP)**
• **IIZUKA, Mariko**
  **Saitama 344-0117 (JP)**
• **IKEDA, Katsumi**
  **Ashiya-shi, Hyogo 6590064 (JP)**
• **YAMAMOTO, Junko**
  **Kyoto-shi, Kyoto 6060832 (JP)**

(74) Representative: **Kalhammer, Georg et al**
**Lederer & Keller**
**Patentanwälte**
**Prinzregentenstrasse 16**
**80538 München (DE)**

(54) **ALGA EXTRACT AND LIPASE INHIBITOR CONTAINING THE SAME**

(57) A lipase inhibitor comprising as an active ingredient an extract of Ascophyllum nodosum which is a kind of brown algae can be used as a useful healthy food or food for specified health uses for the treatment and/or prevention of obesity or hyperlipemia.

EP 1 693 084 A1

**Description**

TECHNICAL FIELD

The present invention relates to a lipase inhibitor containing a marine algae extract as an active ingredient, more particularly, an extract of Ascophyllum nodosum which is a kind of brown algae, and also relates to a food and drink for the treatment and/or prevention of obesity and hyperlipemia.

BACKGROUND ART

Recently, with westernization of eating habits, obesity is increasing due to hypernutrition and the like. Obesity is one of risk factors of arteriosclerosis and concerned also with diabetes, hypertension and the like, thus, which has become a serious social problem. Obesity is a condition where fats are accumulated in excess in the body, and one of the causes is an excessive intake of fats.

In general, an excessive intake of calories works to increase stored calories, and as a result, stored calories increase in the body. That is, excessive intake of a fat of highest calorie among food components leads to obesity. Then, it is believed that obesity can be prevented or ameliorated by inhibiting a pathway from fat intake to obesity.

Fat in foods is not absorbed in its original form from the intestinal canal. Namely, the fat (triglyceride) is degraded by pancreatic lipase into fatty acid, and 2-monoglyceride or glycerol, which are then absorbed from the intestine. In the intestine, these are resynthesized and transported into blood. Therefore, degradation of fat is suppressed, and absorption of fat from the intestinal canal is also suppressed, by inhibiting the activity of pancreatic lipase.

As such a lipase inhibitor, Orlistat (trade name: Zenical, Roche) is practically used abroad clinically as a medicine (see, e.g., non-patent literature 1: Ikeda Yoshio, "Recent use rate of Orlistat abroad", Himan Kenkyu, Nippon Obesity Institute, 2001, vol. 7, no. 3, p. 316-318).

However, this product is currently not accepted to be used in Japan, and cannot be used not only as a medicine but also as a food under the present situation.

A lipase inhibitor and a food containing this substance are useful for a patient suffering from related dysbolism since it can ameliorate pathological condition of the above-mentioned disease, and further, it is suitable also for prevention of obesity and hyperlipemia by taking into daily eating habits. Therefore, as a highly safe natural substance which is edible, there have hitherto been suggested lipase inhibitors derived from marine algae (see, e.g., patent literatures 1, 2 and 3: JP-A-5-284937, JP-A-10-203974, and JP-A-2000-236846), green pepper, pumpkin, shimeji mushroom, Glifola frondosa, Hizikia fusiforme, others (see, e.g., patent literature 4: JP-A- 3-219872 ), Labiatae (see, e.g. patent literature 5: JP-A-10-262606), hop (see, e.g., patent literature 6: JP-A-2001-321166), defatted rice bran (see, e.g., patent literature 7: JP-A-2001-97880), tamarind seed coat (see, e.g., patent literature 8: JP-A- 9-291039) and the like.

On the other hand, Ascophyllum nodosum is a marine algae belonging to brown algae, Fucales, Fucaceae, and mainly inhabits on the shore reef of ria shoreline in Norway. Ascophyllum nodosum is used as a raw material for producing alginic acid, because it contains alginic acid in high concentration. In addition, since Ascophyllum nodosum contains abundantly minerals and vitamins, a product obtained by drying of a raw alga and pulverization thereof is widely used as a feedstuff or a fertilizer and/or a soil improving agent. However, an extract of Ascophyllum nodosum has not been known to have an inhibitory action on lipase activity.

DISCLOSURE OF THE INVENTION

It is an object of the present invention to provide a lipase inhibiting substance derived from natural products, which inhibits pancreatic lipase as an enzyme involved in digestion and absorption of fats in an organism to suppress accumulation of fats in the body, and to provide a lipase inhibitor containing the above inhibiting substance.

The present inventors have intensively studied to solve the above-mentioned problem, and as a result, have found that an extract of Ascophyllum nodosum which is a kind of brown algae has a strong inhibitory action on pancreatic lipase, and further have found that this extract has also an action of lowering the plasma triglyceride, namely, has an action of decreasing the amount of triglyceride in plasma as well. The present inventors have further studied based on these findings, and completed the present invention.

That is, the present invention relates to:

(1) a lipase inhibitor comprising an extract of Ascophyllum nodosum as an active ingredient,
(2) a lipase inhibitor comprising a purified substance of the extract according to the above (1) as an active ingredient,
(3) the lipase inhibitor according to the above (1) or (2), which is in the form of food and drink,
(4) the lipase inhibitor according to the above (3), which is in the form of healthy food or food for specified health uses for the treatment and/or prevention of obesity,

(5) the lipase inhibitor according to the above (3), which is in the form of healthy food or food for specified health uses for the treatment and/or prevention of hyperlipemia,

(6) a plasma triglyceride-lowering agent comprising an extract of Ascophyllum nodosum as an active ingredient,

(7) a method of inhibiting lipase activity, which comprises administering an extract of Ascophyllum nodosum to a mammal,

(8) a method of treating and/or preventing obesity or hyperlipemia, which comprises administering an extract of Ascophyllum nodosum to a mammal,

(9) a method of lowering plasma triglyceride, which comprises administering an extract of Ascophyllum nodosum to a mammal,

(10) use of an extract of Ascophyllum nodosum for producing a medicine or food and drink which inhibits lipase activity,

(11) use of an extract of Ascophyllum nodosum for producing a medicine or food and drink for treating and/or preventing obesity or hyperlipemia, and

(12) use of an extract of Ascophyllum nodosum for producing a medicine or food and drink which lowers plasma triglyceride.

EFFECT OF THE INVENTION

The extract from Ascophyllum nodosum obtained in the present invention has a strong lipase inhibiting action and a strong plasma triglyceride lowering active action. Thus, a lipase inhibitor or triglyceride lowering agent comprising the above-mentioned extract can treat and/or prevent obesity and hyperlipemia more effectively as compared with conventionally known lipase inhibiting substances derived from marine algae.

The lipase inhibitor of the present invention can treat and/or prevent obesity and hyperlipemia. Thus, the lipase inhibitor is useful for patients suffering from dysbolism related to obesity and hyperlipemia, and can be incorporated in food and drink, particularly in healthy food or food for specified health uses in daily eating habits.

BEST MODES FOR CARRYING OUT THE INVENTION

In the present invention, any tissues and portions of Ascophyllum nodosum (hereinafter, abbreviated as Ascophyllum), preferably leaf and stem parts of algae can be used. In extracting from Ascophyllum, total algae or leaf and stem parts of Ascophyllum harvested from the sea can be used as they are, or they can be cut, finely cut or ground, or dried them. Furthermore, total algae or leaf and stem parts of algae which is cut, finely cut or grounded after drying can be used. Preferably, total algae or leaf and stem parts of raw Ascophyllum which is grounded after drying can be used. Drying may be carried out by any methods known per se, for example, air drying, sun drying, freeze drying and the like.

As the extraction solvent, water or organic solvents, or mixed solutions thereof are used. Examples of the organic solvent include polar organic solvents such as lower alcohols having 1 to 4 carbon atoms such as methanol, ethanol, propanol, isopropanol, n-butanol, isobutanol, sec-butanol, tert-butanol and the like, and ketones such as dimethyl ketone, methyl ethyl ketone, acetone, methyl isobutyl ketone and the like; and non-polar organic solvents such as methyl acetate, ethyl acetate, butyl acetate, diethyl ether and the like. These polar organic solvents and non-polar organic solvents can also be used in appropriate combination.

Of these extraction solvents, preferrable are polar organic solvents or mixed solutions of polar organic solvents and water, more preferable are methanol, ethanol or acetone or mixed solutions of them and water, and particularly preferable are mixed solutions of methanol, ethanol or acetone and water. The mixing ratio of a polar organic solvent to water varies depending on the kind of a polar organic solvent, and usually, polar organic solvent/water is in a range of about 5/95 to 100/0 (v/v). For example, when a methanol-water mixed solution or ethanol-water mixed solution is used as the extraction solvent, the ratio is about 5/95 to 100/0 (v/v), preferably about 30/70 to 70/30 (v/v). When an acetone-water mixed solution is used, the ratio is about 5/95 to 100/0 (v/v), preferably about 30/70 to 80/20 (v/v). These ratios are preferably determined taking extraction efficiency, amount of extracted substance, enzyme inhibitory activity of extracts, and the like into consideration.

In the present invention, the extraction method for obtaining an extract is not particularly restricted, and methods known per se can be used such as, for example, immersion extraction, heat extraction, continuous extraction, supercritical extraction and the like. The ratio of Ascophyllum to extraction solvent is not particularly limited, and the ratio of dried Ascophyllum substance/solvent is preferably about 1/100 to 1/2 (w/v), more preferably about 1/10 to 1/5 (w/v). Specifically, extraction is preferably carried out while gently stirring or allowing to stand using an extraction solvent in an amount of about 200 mL to 10 L, preferably about 500 mL to 1 L based on about 100 g of the extraction raw material which is obtained by, for example, drying and grinding Ascophyllum. It is convenient in view of operability that the extraction temperature is in a range from room temperature to not higher than the boiling point of the solvent under normal pressure, and the extraction time varies depending on the extraction temperature and the like, and is in a range from several minutes to about 7 days, preferably from about 30 minutes to 24 hours.

After completion of the extraction operation, solid (extraction residue) is removed by methods known per se such as filtration, centrifugation and the like, to obtain an extract. The extract is concentrated by a method known per se, thereby to obtain an extract concentrated in the form of black to brown oil or paste (hereinafter, referred simply to as concentrate in some cases). The extract or the concentrate can also be converted into a solid extract by performing drying known per se such as, for example, thermal drying, freeze drying and the like. An extract, concentrate, or solution obtained by dissolving the concentrate in water and/or organic solvent may be purified by a method such as, for example, ultrafiltration, adsorption resin treatment, molecule chromatography, partition chromatography, liquid-liquid extraction and the like. The purified extract can be used for the present invention in the form of purified substance.

The extract according to the present invention is useful as a lipase inhibitor since it has a strong lipase inhibitory action.

Regarding the lipase inhibitor of the present invention, it is preferable that the above-mentioned extract or purified substance is used as it is, or a pharmaceutically acceptable additive, or a food material, food raw material, further if necessary, a food additive and the like are appropriately mixed with the extract or purified substance, and they are preferably formulated into dosage form such as liquid, powder, granule, tablet, microcapsule, soft capsule, hard capsule and the like by methods known per se. Moreover, it is possible to make into any food and drink forms such as solid food, semisolid food like cream or jam, food like gel, beverage and the like. Examples of such food and drink include refreshing beverage, coffee, tea, milk-contained beverage, lactic acid bacteria beverage, drop, candy, chewing gum, chocolate, gummy candy, yoghurt, ice cream, pudding, soft adzuki-bean jelly, jelly, cookie and the like. These various preparations or foods and drinks are useful as a healthy food or food for specified health uses for the treatment and/or prevention of diabetes.

As the additive, food material, food raw material and food additive used in the production of the above-mentioned preparations or foods and drinks, for example, excipients (lactose, corn starch, white sugar, glucose, starch, crystalline cellulose and the like), lubricants (magnesium stearate, sucrose fatty acid ester and the like), disintegrators (starch, carmellose sodium, calcium carbonate and the like), binders (starch paste liquid, hydroxypropylcellulose liquid, gum Arabic liquid and the like), emulsifiers and/or solubilizers (gum arabic, polysorbate 80, povidone and the like), sweeteners (white sugar, fructose, simple syrup, honey and the like), coloring agents (edible tar pigment, iron oxide and the like), preservatives (methyl p-oxybenzoate, propyl p-oxybenzoate, sorbic acid and the like), thickeners (hydroxyethylcellulose, polyethylene glycol, sodium alginate and the like), antioxidants (sodium hydrogen sulfite, sodium edetate, ascorbic acid and the like), stabilizers (sodium thiosulfate, sodium edentate, sodium citrate and the like), acidulants (lemon juice and the like), seasonings (sodium glutamate and the like), aromatics (mint, strawberry aroma and the like), and the like can be used.

The addition amount of the above-mentioned extract or purified substance based on the above-mentioned various preparations or foods and drinks is not uniform and varies depending on the content of a lipase inhibiting component contained in the extract or purified substance, and the amount of the extract (calculated as the solid) is, for example, about 0.0001 to 50 wt%, preferably about 0.001 to 20 wt%, more preferably about 0.01 to 10 wt%.

When these various preparations or foods or drinks are taken orally, the daily dose of the above-mentioned extract or purified substance is about 0.01 to 1000 mg, preferably about 0.1 to 500 mg, more preferably about 1 to 300 mg relative to 1 kg of body weight when calculated as the solid. This dose may be advantageously taken in one time or several times per day. However, actual dose should be determined in view of the object and conditions of a person who takes it (sex, age, body weight, BMI and the like).

Preferable Examples in the present invention are described below, but the present invention is not limited to these Examples.

Example 1

About 50.0 g of a dried Ascophyllum powder was precisely weighed and to this dried powder was added 500 mL of an ethanol-water mixed solution in a ratio shown Table 1, and extraction was performed for 1 hour at room temperature with gentle stirring. The extraction solution was moved to a centrifuge tube, and divided into a supernatant and a precipitate by centrifugation, and 500 mL of the same ethanol-water mixed solution as described above was added to the precipitate, and extraction was performed for 1 hour in the same manner as in the first operation. The extract was divided into a supernatant and a precipitate in the same manner as in the first operation, and the supernatants of the first and second operations were combined and filtrated under suction, to obtain an extract in a total volume of about 1 L as a filtrate. This extract was concentrated at about 60°C under reduced pressure using a rotary evaporator, and the concentrate was freeze-dried to obtain extracts 1 to 6 in the form of black brown powder. The yields are shown in Table 1.

Table 1

| Extract | Ethanol-water mixed solution (ethanol:water (v/v)) | Yield (% by mass) |
|---|---|---|
| 1 | 10:90 | 24.2 |
| 2 | 20:80 | 24.3 |
| 3 | 30:70 | 24.3 |
| 4 | 50:50 | 22.0 |
| 5 | 70:30 | 17.0 |
| 6 | 100:0 | 2.2 |

Example 2

To about 800 g of a dried Ascophyllum powder was added 8 L of an ethanol-water (50:50 (v/v)) mixed solution, and extraction was performed for 1 hour at room temperature with gentle stirring. The extract was moved to a centrifuge tube, and divided into a supernatant and a precipitate by centrifugation, and 8 L of the ethanol-water mixed solution was added to the precipitate, and extraction was performed for 1 hour in the same manner as in the first operation. The extract was divided into a supernatant and a precipitate in the same manner as in the first operation, and the supernatants of the first and second operations were combined and filtred under suction, to obtain an extract in a total volume of about 16 L as a filtrate. This extract was subjected to ultrafiltration using an ultrafiltration membrane having a fractional molecular weight of 10,000 (trade name: FB02-VC-FUSO181; Daicen Membrane Systems), and when the concentrated solution reached a volume of 5 L, 5 L of water was added thereto and filtration was continued, and when the concentrated solution reached again a volume of 5 L, ultrafiltration was stopped. The concentrated solution was concentrated at about 60°C under reduced pressure using a rotary evaporator, and the concentrate was freeze-dried to obtain about 73 g of an extract (extract 7) in the form of black brown powder.

Example 3

The lipase inhibitory activity of the extract 7 obtained in Example 2 was measured using triolein as a substrate.

1) Measurement of lipase inhibiting activity

Sample solutions (1 mL each) containing the extract 7 in an amount of 5, 10, 50, 100 and 500 $\mu$g/mL respectively, 1 mL of 1 mg/mL lipase (Type II; Sigma) solution (pH 7.4), 7 mL of Mcilvaine buffer solution (pH 7.4), 100 mg of gum arabic, and 1.0 mg of triolein (manufactured by Wako Pure Chemicals Industries Ltd.) were mixed, and shaken at about 37°C for 1 hour, and 20 mL of ethanol was added to stop the reaction, thereby to obtain the reaction solution. In control group 1, 1 mL of Mcilvaine buffer solution (pH 7.4) was added instead of the lipase solution, and in control group 2, 1 mL of Mcilvaine buffer solution (pH 7.4) was added instead of the sample solution. To the reaction solution were added several drops of a phenolphthalein solution, and the reaction solution was titrated with 0.05 N NaOH, and the lipase inhibition rate was calculated according to the following equation.

$$\text{Lipase inhibition rate (\%)} = (C-S)/(C-B) \times 100$$

S: titration amount in test sample group (mL)
B: titration amount in control group 1 (mL)
C: titration amount in control group 2 (mL)

The lipase inhibition rate of the extract was measured and the results are shown in Table 2.

Table 2

| Concentration of extract 7 ($\mu$g/mL) | Lipase inhibition rate (%) |
|---|---|
| 5 | 9.2 |
| 10 | 15.4 |

(continued)

| Concentration of extract 7 ($\mu$g/mL) | Lipase inhibition rate (%) |
|---|---|
| 50 | 67.7 |
| 100 | 80.0 |
| 500 | 90.8 |

The 50% inhibitory concentration ($IC_{50}$) of the extract 7 in Example 2 on lipase activity was found to be about 29 $\mu$g/mL which was calculated based on the above results.

Example 4

About 50.0 g of dried powders of various marine algae were precisely weighed and to these dried powders was added each 500 mL of an ethanol-water (30:70 (v/v)) mixed solution. The mixture was extracted for 1 hour at room temperature with gentle stirring. The extract was transferred to a centrifuge tube, and divided into a supernatant and a precipitate by centrifugation. 500 mL of the ethanol-water (30:70 (v/v)) mixed solution was added to the precipitate, and the mixture was extracted with for 1 hour in the same manner as in the first operation. The extract was divided into a supernatant and a precipitate in the same manner as in the first operation, and the supernatants of the first and second operations were combined and filtered under suction, thereby to obtain an extract in a total volume of about 1 L as a filtrate. This extract was concentrated at about 60°C under reduced pressure using a rotary evaporator, and the concentrate was freeze-dried to obtain extracts (extract 8, Comparative Examples 1 to 8) in the form of powder.
The lipase inhibition activity of these extracts was measured according to Example 3 described above. The concentration of the sample solution was 100 $\mu$g/mL or 1000 $\mu$g/mL. The results are shown in Table 3 in terms of inhibition rate (%).

Table 3

| | Kind of marine algae | Lipase inhibition rate (%) | |
|---|---|---|---|
| | | 100 $\mu$g/mL | 1000 $\mu$g/mL |
| Extract 8 | Ascophyllum nodosum (brown algae) | 40.6 | 96.9 |
| Comparative Example 1 | Ulva pertusa Kjellman (green algae) | 0 | 0 |
| Comparative Example 2 | Nemacystus decipiens (brown algae) | 0 | 0 |
| Comparative Example 3 | Laminaria japonica (brown algae) | 0 | 34.3 |
| Comparative Example 4 | Eisenia bicyclis (Kjellman) Setchell (brown algae) | 7.2 | 25.0 |
| Comparative Example 5 | Undaria pinnatifida (brown algae) | 4.5 | 20.0 |
| Comparative Example 6 | Sargassum fulvellum (brown algae) | 5.8 | 27.1 |
| Comparative Example 7 | Hizikia fusiformis (brown algae) | 0 | 0 |
| Comparative Example 8 | Ptilophora subcostata (red algae) | 0 | 0 |

From Table 3, it is shown that an extract of Ascophyllum has a stronger lipase inhibition activity as compared with other marine algae, and additionally, the inhibitory activity is exhibited even at lower concentrations.

Example 5

Single administration test of oil was performed in mice using the extract 7 obtained in Example 2 as a sample.

1) Measurement of blood triglyceride lowering activity

Each eight 7-week old ddY mice fasted overnight were used in a control group and a sample administration group. About 50 $\mu$L of blood was collected from the mouse tail vein into a heparin-containing blood collection tube. After blood collection, an emulsion of olive oil and physiological saline so prepared as to give olive oil 5 g/body weight kg/6 mL was orally administered using a gastric tube, in the control group. Emulsions of olive oil and a sample solution dissolved in physiological saline so mixed and prepared that the olive oil was 5 g/body weight kg/6 mL and the extract 7 as a sample was 100 mg/body weight kg/6 mL or 500 mg/body weight kg/6 mL, were orally administered using a gastric sonde, in the sample administration group. About 50 μL of blood was collected 1, 2, 3, 4 and 5 hours after administration. The collected blood was centrifuged to fractionate a plasma fraction, and preserved at -40°C until analysis.

The plasma level of triglyceride was measured by GPO/DASO method using a measuring kit (Triglyceride E - Test Wako; manufactured by Wako Pure Chemical Industries Ltd.). The change with time of the plasma triglyceride level is shown in Table 4.

Table 4

| Elapsed time (hour) | Plasma level of triglyceride (mg/100 mL) (numerical value is average value ± standard deviation) | | |
| --- | --- | --- | --- |
| | Control group | Extract 7 administration group | |
| | | 100 mg/body weight kg | 500 mg/body weight kg |
| 0 | 336 ± 103 | 258 ± 62 | 267 ± 87 |
| 1 | 550 ± 147 | 514 ± 76 | 255 ± 81 ** |
| 2 | 1000 ± 284 | 781 ± 189 | 358 ± 126 ** |
| 3 | 1062 ± 300 | 747 ± 215 * | 265 ± 83 ** |
| 4 | 78 ± 417 | 652 ± 229 | 180 ± 59 ** |
| 5 | 451 ± 356 | 413 ± 264 | 147 ± 26 ** |
| ** significant difference with a crisis ratio of 1% for the control group<br>* significant difference with a crisis ratio of 5% for the control group | | | |

It was found that an extract of Ascophyllum suppresses significantly increase in the plasma level of triglyceride 1 to 5 hours after administration as compared with the control group.

Example 6

To 50 parts by mass of lactose, 38 parts by mass of corn starch, 1 part by mass of strawberry aroma and 1 part by mass of sucrose fatty acid ester were added 10 parts by mass of the extract 7 in Example 2 and they were mixed, and then the mixture was tabletted using a tabletting machine to produce a supplement.

Example 7

A beverage solution having the composition shown in Table 5 was heated at about 65°C for 10 minutes, cooled to room temperature, and filled aseptically in a sterile container to produce a honey lemon beverage.

Table 5

| Component | Addition amount (% by mass) |
| --- | --- |
| Fructose-glucose syrup | 11 |
| Lemon juice | 3 |
| Honey | 1 |
| Aroma | 0.1 |
| Acidulant | 0.1 |
| Vitamin C | 0.02 |
| Pigment | 0.01 |
| Extract 7 of Example 2 | 1.00 |
| Water | 84.77 |
| Total | 100 |

Example 8

Orange jelly was produced according to the following procedure.

(1) 15 g of gelatin powder is placed in about 45 mL of water and swollen.
(2) 750 mL of orange juice (fruit juice 100%) and 90 g of granulated sugar are put in a pan and boiled. When the granulated sugar is dissolved, fire is extinguished, and about 7 g of the extract 7 in Example 2 and (1) are added and dissolved sufficiently.
(3) Ice water is allowed to contact the bottom of the pan and the content is cooled while mixing until thickened, and poured into a jelly mold of which inside wall has been moistened, and cooled to solidify.

INDUSTRIAL APPLICABILITY

An extract from Ascophyllum nodosum obtained in the present invention has a strong lipase inhibition action and a strong plasma triglyceride lowering action. Therefore, a lipase inhibitor containing the extract from Ascophyllum nodosum can be used for more effective treatment and/or prevention of obesity and hyperlipemia as compared with conventionally known lipase- inhibiting substances derived from marine algae. Moreover, a food and drink containing the above-mentioned extract is useful as a healthy food or food for specified health uses for the treatment and/or prevention of obesity and hyperlipemia.

**Claims**

1.    A lipase inhibitor comprising an extract of Ascophyllum nodosum as an active ingredient.

2.    A lipase inhibitor comprising a purified substance of the extract according to claim 1 as an active ingredient.

3.    The lipase inhibitor according to claim 1 or 2, which is in the form of food and drink.

4.    The lipase inhibitor according to claim 3, which is in the form of healthy food or food for specified health uses for the treatment and/or prevention of obesity.

5.    The lipase inhibitor according to claim 3, which is in the form of healthy food or food for specified health uses for the treatment and/or prevention of hyperlipemia.

6.    A plasma triglyceride-lowering agent comprising an extract of Ascophyllum nodosum as an active ingredient.

7.    A method of inhibiting lipase activity, which comprises administering an extract of Ascophyllum nodosum to a mammal.

8.    A method of treating and/or preventing obesity or hyperlipemia, which comprises administering an extract of Ascophyllum nodosum to a mammal.

9.    A method of lowering plasma triglyceride, which comprises administering an extract of Ascophyllum nodosum to a mammal.

10.    Use of an extract of Ascophyllum nodosum for producing a medicine or food and drink which inhibits lipase activity.

11.    Use of an extract of Ascophyllum nodosum for producing a medicine or food and drink for treating and/or preventing obesity or hyperlipemia.

12.    Use of an extract of Ascophyllum nodosum for producing a medicine or food and drink which lowers plasma triglyceride.

<div align="center">

**INTERNATIONAL SEARCH REPORT**

</div>

| | International application No. |
|---|---|
| | PCT/JP2004/018369 |

**A. CLASSIFICATION OF SUBJECT MATTER**
Int.Cl⁷ A61K35/80, A61P3/04, 3/06, 43/00, A23L1/30, 2/00, C12N9/99

According to International Patent Classification (IPC) or to both national classification and IPC

**B. FIELDS SEARCHED**

Minimum documentation searched (classification system followed by classification symbols)
Int.Cl⁷ A61K35/80, A61P3/04, 3/06, 43/00, A23L1/30, 2/00, C12N9/99

Documentation searched other than minimum documentation to the extent that such documents are included in the fields searched

Electronic data base consulted during the international search (name of data base and, where practicable, search terms used)
CA(STN), BIOSIS(STN), MEDLINE(STN), EEMBASE(STN), JICST(JOIS)

**C. DOCUMENTS CONSIDERED TO BE RELEVANT**

| Category* | Citation of document, with indication, where appropriate, of the relevant passages | Relevant to claim No. |
|---|---|---|
| X | JP 2003-160505 A (Lion Corp.), 03 June, 2003 (03.06.03), (Family: none) | 4,11 |
| X | JP 2000-72642 A (Lion Corp.), 07 March, 2000 (07.03.00), (Family: none) | 4,11 |
| A | WO 2002/22140 A1 (Takara Bio Inc.), 21 March, 2002 (21.03.02), Claim 6; page 6, line 27 & AU 880400 A & EP 1327448 A1 & US 2004/29828 A1 | 1-6,10-12 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 27 December, 2004 (27.12.04) | Date of mailing of the international search report 18 January, 2005 (18.01.05) |
|---|---|
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2004/018369 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 5-284937 A  (Kabushiki Kaisha TAC Gijutsu Kagaku Kenkyusho), 01 November, 1993 (01.11.93), Table 3 (Family: none) | 1-6,10-12 |
| A | Dalin Ren et al., Study on Antihypertensive and Antihyperlipidemic Effects on Marine Algae, Fisher Science, 1994, Vol.60, No.1, pages 83 to 88, table 4 | 1-6,10-12 |

Form PCT/ISA/210 (continuation of second sheet) (January 2004)

## INTERNATIONAL SEARCH REPORT

| International application No. |
|---|
| PCT/JP2004/018369 |

**Box No. II     Observations where certain claims were found unsearchable (Continuation of item 2 of first sheet)**

This international search report has not been established in respect of certain claims under Article 17(2)(a) for the following reasons:

1. ☒ Claims Nos.: 7 – 9
    because they relate to subject matter not required to be searched by this Authority, namely:
    The inventions as set forth in claims 7 to 9 pertain to methods for treatment of the human body by therapy.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest.

                                    ☐ No protest accompanied the payment of additional search fees.

Form PCT/ISA/210 (continuation of first sheet (2)) (January 2004)